(19) **Europäisches Patentamt**
**European Patent Office**
**Office européen des brevets**

(11) **EP 4 537 086 B1**

(12) **FASCICULE DE BREVET EUROPEEN**

(45) Date de publication et mention
de la délivrance du brevet:
**05.08.2026 Bulletin 2026/32**

(51) Classification Internationale des Brevets (IPC):
**G01N 33/00** (2006.01)   **G01N 21/45** (2006.01)
**G01N 21/552** (2014.01)

(21) Numéro de dépôt: **23733800.9**

(22) Date de dépôt: **01.06.2023**

(52) Classification Coopérative des Brevets (CPC):
**G01N 33/0031; G01N 21/45; G01N 21/553;
G01N 2201/12753**

(86) Numéro de dépôt international:
**PCT/IB2023/055646**

(87) Numéro de publication internationale:
**WO 2023/237980 (14.12.2023 Gazette 2023/50)**

(54) **PROCÉDÉ ET PROGRAMME D'ORDINATEUR POUR L'ÉTALONNAGE D'UN DISPOSITIF ÉLECTRONIQUE DE CARACTÉRISATION D'UN FLUIDE, DISPOSITIF ÉLECTRONIQUE CORRESPONDANT**

VERFAHREN UND COMPUTERPROGRAMM ZUR KALIBRIERUNG EINER ELEKTRONISCHEN VORRICHTUNG ZUR CHARAKTERISIERUNG EINES FLUIDS UND ENTSPRECHENDE ELEKTRONISCHE VORRICHTUNG

METHOD AND COMPUTER PROGRAM FOR CALIBRATING AN ELECTRONIC DEVICE FOR CHARACTERIZING A FLUID, AND CORRESPONDING ELECTRONIC DEVICE

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **10.06.2022 FR 2205630**

(43) Date de publication de la demande:
**16.04.2025 Bulletin 2025/16**

(73) Titulaire: **Olfaktion**
**75017 Paris (FR)**

(72) Inventeurs:
• **NGUYEN, Van Tri**
**38000 Grenoble (FR)**
• **CARITU, Yanis**
**38000 Grenoble (FR)**
• **MAHO, Pierre**
**38000 Grenoble (FR)**

(74) Mandataire: **Bonnet, Michel**
**Cabinet Bonnet**
**93, rue Réaumur - Boîte 10**
**75002 Paris (FR)**

(56) Documents cités:
**WO-A1-00/42907**          **FR-A1- 3 107 120**
**US-A1- 2020 132 644**

• **LEI ZHANG ET AL: "On-line sensor calibration transfer among electronic nose instruments for monitoring volatile organic chemicals in indoor air quality", SENSORS AND ACTUATORS B: CHEMICAL, ELSEVIER BV, NL, vol. 160, no. 1, 30 August 2011 (2011-08-30), pages 899 - 909, XP028110983, ISSN: 0925-4005, [retrieved on 20110908], DOI: 10.1016/J.SNB.2011.08.079**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne un procédé d'étalonnage d'un dispositif électronique de caractérisation d'un fluide à au moins un capteur conçu pour interagir avec le fluide, par comparaison avec un dispositif électronique de caractérisation de référence. Elle concerne un programme d'ordinateur correspondant, ainsi qu'un dispositif électronique étalonné de caractérisation d'un fluide par la fourniture d'une signature obtenue à partir d'un signal électrique de mesure.

L'invention s'applique plus particulièrement à un procédé d'étalonnage comportant les étapes suivantes :

- stockage en mémoire d'une pluralité de signatures d'étalonnage de référence relatives respectivement à une interaction d'une pluralité de fluides d'étalonnage prédéterminés avec au moins un capteur du dispositif électronique de référence ;

- stockage en mémoire d'un modèle paramétrique, à au moins un paramètre réglable, de dérive du dispositif électronique à étalonner par rapport au dispositif électronique de référence ;

- pour chacun de la pluralité de fluides d'étalonnage prédéterminés, obtention d'une signature d'étalonnage par interaction avec ledit au moins un capteur du dispositif électronique à étalonner ;

- détermination d'au moins une valeur dudit au moins un paramètre réglable par comparaison des signatures d'étalonnage obtenues avec les signatures d'étalonnage de référence ;

Ainsi, en appliquant ladite au moins une valeur déterminée de paramètre réglable dans le modèle paramétrique de dérive, il est possible de corriger toute signature obtenue par interaction d'un fluide quelconque avec ledit au moins un capteur du dispositif électronique tel qu'étalonné de cette manière.

Un tel procédé d'étalonnage est par exemple enseigné dans l'article de Zhang et al, intitulé « On-line sensor calibration transfer among electronic nose instruments for monitoring volatile organic chemicals in indoor air quality », publié dans Sensors and Actuators B: Chemicals, volume 160, issue 1, pages 899-909, le 15 décembre 2011. Dans cet article, c'est un modèle de dérive par transformation affine globale qui est retenu sur la base d'une hypothèse d'homogénéité linéaire entre dispositifs électroniques de caractérisation olfactive de fluides. L'hypothèse s'avère pertinente, mais la réalité des dérives ne suit malheureusement pas tout à fait le modèle.

Pour affiner le modèle paramétrique de dérive, il est possible de le rendre plus complexe. Mais cela rend l'étalonnage également plus compliqué

Il peut ainsi être souhaité de prévoir un procédé d'étalonnage qui permette de s'affranchir d'au moins une partie des problèmes et contraintes précités.

Selon la revendication indépendante 1, il est donc proposé un procédé d'étalonnage d'un dispositif électronique de caractérisation d'un fluide à au moins un capteur conçu pour interagir avec le fluide, par comparaison avec un dispositif électronique de caractérisation de référence, comportant les étapes suivantes :

- stockage en mémoire d'une pluralité de signatures d'étalonnage de référence relatives respectivement à une interaction d'une pluralité de fluides d'étalonnage prédéterminés avec au moins un capteur du dispositif électronique de référence ;

- stockage en mémoire d'un modèle paramétrique, à au moins un paramètre réglable, de dérive du dispositif électronique à étalonner par rapport au dispositif électronique de référence ;

- pour chacun de la pluralité de fluides d'étalonnage prédéterminés, obtention d'une signature d'étalonnage par interaction avec ledit au moins un capteur du dispositif électronique à étalonner ;

- détermination d'au moins une valeur dudit au moins un paramètre réglable par comparaison des signatures d'étalonnage obtenues avec les signatures d'étalonnage de référence ;

dans lequel les fluides d'étalonnage prédéterminés sont répartis en plusieurs groupes d'étalonnage différents et la détermination de ladite au moins une valeur dudit au moins un paramètre réglable est réalisée spécifiquement pour chaque groupe d'étalonnage.

Ainsi, en prévoyant astucieusement plusieurs groupes d'étalonnage différents pour lesquels les valeurs respectives de paramètres du modèle paramétrique de dérive sont spécifiques, donc *a priori* différentes d'un groupe d'étalonnage à l'autre, le procédé d'étalonnage est rendu plus fin, plus proche de la réalité des dérives possibles, sans pour autant avoir besoin de compliquer le modèle paramétrique de dérive à utiliser. Des améliorations substantielles ont d'ailleurs été mesurées expérimentalement. Il convient en outre de noter que ce procédé d'étalonnage s'applique aussi bien pour la dérive dans le temps d'un même dispositif électronique de caractérisation de fluide, i.e. la dérive en répétabilité de ce dispositif, que pour la dérive en fabrication de plusieurs dispositifs électroniques de caractérisation de fluide, i.e. la dérive en reproductibilité de ces dispositifs.

De façon optionnelle, un procédé d'étalonnage selon l'invention peut en outre comporter une étape d'application de ladite au moins une valeur de paramètre réglable dans le modèle paramétrique de dérive pour corriger toute signature obtenue par interaction d'un fluide quelconque avec ledit au moins un capteur du dispositif électronique à étalonner, cette étape comportant la sélection d'un groupe d'étalonnage à associer au fluide quelconque en fonction de sa signature, puis la correction de sa signature à l'aide de ladite au moins une valeur de paramètre réglable du groupe sélectionné. Il est en effet astucieux d'associer, au principe précité d'étalonnage par groupes différenciés, une étape de sélection préalable de groupe d'étalonnage pour tout fluide à caractériser sur la base de sa signature avant même de corriger cette signature par étalonnage en prenant en compte le modèle de dérive avec les spécificités du groupe sélectionné.

De façon optionnelle également, la sélection d'un groupe d'étalonnage à associer au fluide quelconque comporte une classification automatique de sa signature dans l'un des groupes d'étalonnage par comparaison de cette signature avec la ou les signature(s) d'étalonnage obtenue(s) du ou des fluide(s) d'étalonnage de chaque groupe d'étalonnage.

De façon optionnelle également, la correction de la signature du fluide quelconque comporte une inversion du modèle paramétrique de dérive dans lequel est appliquée ladite au moins une valeur de paramètre réglable du groupe d'étalonnage sélectionné.

De façon optionnelle également, l'obtention d'une signature d'étalonnage pour chacun de la pluralité de fluides d'étalonnage prédéterminés comporte l'obtention d'une première signature d'étalonnage à N composante(s), $N \geq 1$, puis la transformation de cette première signature d'étalonnage par normalisation et/ou réduction de composantes.

De façon optionnelle également, le modèle paramétrique de dérive est un modèle affine à deux paramètres réglables, $YDEV_{r,i} = \alpha_r \cdot YREF_{r,i} + t_r$, où $YDEV_{r,i}$ est la signature d'étalonnage obtenue du fluide d'étalonnage prédéterminé d'indice i du groupe d'étalonnage d'indice r, $YREF_{r,i}$ est la signature d'étalonnage de référence du fluide d'étalonnage prédéterminé d'indice i du groupe d'étalonnage d'indice r, $\alpha_r$ est la valeur d'un premier paramètre multiplicatif réglable du modèle affine pour le groupe d'étalonnage d'indice r et $t_r$ est la valeur d'un deuxième paramètre additif réglable du modèle affine pour le groupe d'étalonnage d'indice r.

De façon optionnelle également, la correction de la signature du fluide quelconque comporte le calcul $YCOR = (YDEV - t_{\hat{r}})/\alpha_{\hat{r}}$, où $YDEV$ est la signature obtenue par interaction du fluide quelconque avec ledit au moins un capteur du dispositif électronique à étalonner, $YCOR$ est la signature du fluide quelconque après correction et $\hat{r}$ est l'indice du groupe d'étalonnage sélectionné.

De façon optionnelle également, l'un des premier paramètre multiplicatif réglable et deuxième paramètre additif réglable est indépendant des groupes d'étalonnage.

Il est également proposé un programme d'ordinateur selon la revendication indépendante 9.

Selon la revendication indépendante 10, il est également proposé un dispositif électronique étalonné de caractérisation d'un fluide par la fourniture d'une signature obtenue à partir d'un signal électrique de mesure, comportant :

- au moins un capteur conçu pour interagir avec le fluide ;

- un transducteur conçu pour fournir, en interaction avec ledit au moins un capteur, le signal électrique de mesure ;

- une mémoire de stockage :

    • d'un modèle paramétrique, à au moins un paramètre réglable, de dérive du dispositif électronique étalonné par rapport à un dispositif électronique de caractérisation de référence,

    • d'au moins une valeur dudit au moins un paramètre réglable, calculée par un étalonnage préalable du dispositif électronique étalonné par comparaison avec le dispositif électronique de référence sur la base d'une pluralité de fluides d'étalonnage prédéterminés ; et

- un processeur de correction de la signature par application de ladite au moins une valeur de paramètre réglable ;

dans lequel :

- les fluides d'étalonnage prédéterminés sont répartis en plusieurs groupes d'étalonnage différents et la mémoire stocke au moins une valeur spécifique dudit au moins un paramètre réglable pour chacun des groupes d'étalonnage ; et

- le processeur est plus précisément programmé pour sélectionner un groupe d'étalonnage à associer au fluide à caractériser en fonction de sa signature, puis corriger sa signature à l'aide de ladite au moins une valeur spécifique de paramètre réglable du groupe sélectionné.

L'invention sera mieux comprise à l'aide de la description qui va suivre, donnée uniquement à titre d'exemple et faite en se référant aux dessins annexés dans lesquels :

- la figure 1 représente schématiquement la structure générale d'un dispositif électronique étalonné de caractérisation d'un fluide, selon un premier mode de réalisation de l'invention,

- la figure 2 illustre un exemple de superposition de diagrammes temporels de signaux de réponse, ou sensorgrammes, pouvant être obtenus par le dispositif de la figure 1,

- la figure 3 illustre, sous la forme d'un diagramme circulaire, un exemple de signature olfactive normée pouvant être calculée par le dispositif de la figure 1 à partir de sensorgrammes tels que ceux de la figure 2,

- la figure 4 représente schématiquement la structure générale d'un dispositif électronique étalonné de caractérisation d'un fluide, selon un deuxième mode de réalisation de l'invention,

- la figure 5 illustre un exemple de superposition de diagrammes temporels de signaux de réponse, ou sensorgrammes, pouvant être obtenus par le dispositif de la figure 4,

- la figure 6 illustre les étapes successives d'un procédé d'étalonnage pour l'obtention du dispositif électronique étalonné de la figure 1 ou 4, selon un mode de réalisation de l'invention,

- la figure 7 illustre un diagramme de résultats expérimentaux pouvant être obtenus sans appliquer d'étalonnage selon les principes généraux de la présente invention, et

- la figure 8 illustre un diagramme de résultats expérimentaux pouvant être obtenus en appliquant un étalonnage selon les principes généraux de la présente invention.

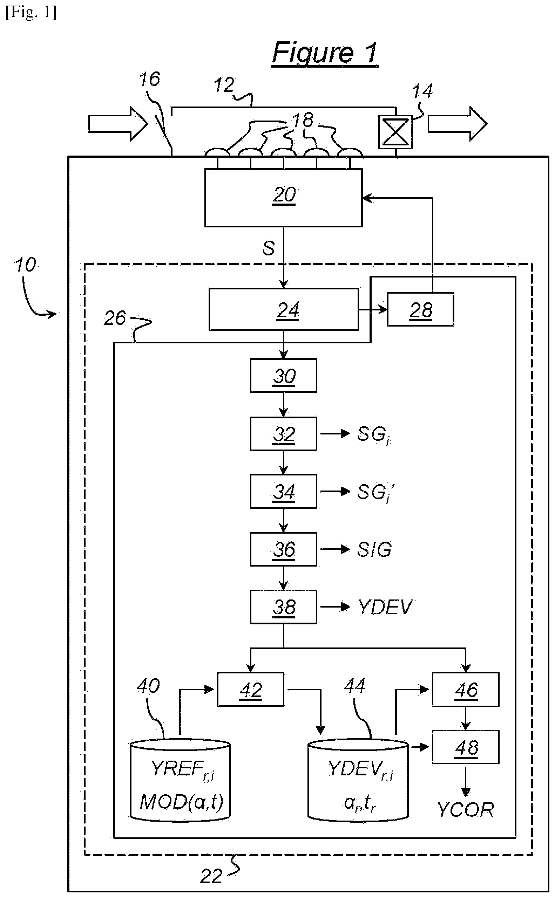

Le dispositif électronique 10 de caractérisation olfactive d'un fluide par la fourniture d'une signature SIG obtenue à partir d'un signal électrique S de mesure, tel que représenté schématiquement sur la figure 1, est un exemple non limitatif de dispositif électronique de caractérisation de fluide selon un premier mode de réalisation de la présente invention pour une application non limitative d'identification d'odeurs par mesure olfactive multivaluée. Il comporte une chambre de mesure 12 destinée à recevoir un fluide, par exemple un gaz tel que de l'air ambiant. Pour ce faire, il comporte un dispositif d'aspiration 14 conçu pour aspirer l'air situé à l'intérieur de la chambre de mesure 12 et le faire sortir à l'extérieur. Il comporte en outre une entrée d'air 16 pouvant être sélectivement fermée pour garder l'air ambiant dans la chambre de mesure 12 ou bien ouverte pour permettre l'évacuation de l'air ambiant de la chambre de mesure 12 et son renouvellement par l'activation du dispositif d'aspiration 14. Il est ainsi doté de moyens de maîtrise des flux entrant et sortant.

Dans sa chambre de mesure 12, Le dispositif électronique de caractérisation olfactive 10 comporte plusieurs capteurs, notamment des capteurs olfactifs 18, répartis respectivement sur autant de sites réactifs, par exemple une soixantaine, conçus pour interagir avec des composés susceptibles d'être présents dans la chambre de mesure 12 lorsque le dispositif 10 est placé à proximité d'un fluide à analyser émetteur de ces composés, notamment lorsque l'entrée d'air 16 est à proximité du fluide considéré. Les composés émis sont généralement des composés organiques volatils mais la présente invention ne se limite pas à de tels composés.

Chaque capteur olfactif 18 est lui-même par exemple un biocapteur conçu pour interagir avec les composés d'une famille particulière de composés organiques volatils. En pratique, chaque capteur olfactif 18 peut comporter une molécule, telle qu'un peptide immobilisé sur un substrat ou un polymère recouvrant une surface, complémentaire des composés de la famille associée à ce capteur olfactif 18.

En variante, le dispositif électronique de caractérisation olfactive 10 pourrait être adapté pour être mis en contact avec tout autre fluide, liquide ou gazeux, que de l'air ambiant. Il pourrait également selon une version particulièrement simple ne pas comporter le dispositif d'aspiration 14 et l'entrée d'air 16, voire même la chambre de mesure 12. Dans cette version simple, les capteurs olfactifs 18 sont alors susceptibles d'être directement mis au contact du fluide à analyser sans maîtrise de flux.

Les capteurs olfactifs 18 sont associés à au moins un transducteur 20 avec lequel ils interagissent. Ce transducteur 20 est arrangé et configuré pour mesurer tout changement de propriété physique provoqué par une interaction des capteurs olfactifs 18 avec le fluide à analyser. Il fournit le signal électrique S de mesure qui se présente par exemple sous la forme d'une séquence de signaux électriques de mesure et caractérise ce fluide puisque cette séquence est représentative des composés organiques volatils avec lesquels les capteurs olfactifs 18 peuvent interagir dans la chambre de mesure 12.

Plus précisément, le transducteur 20 peut être un système d'imagerie par résonance de plasmons de surface, c'est-à-dire un système d'imagerie de type SPR (de l'anglais « Surface Plasmonic Resonance ») configuré pour mesurer tout changement d'un indice de réfraction dû à une interaction du fluide étudié avec au moins l'un quelconque des capteurs olfactifs grâce à un effet plasmonique. Un tel transducteur comporte : une couche métallique dont une première face à sites réactifs sert de support aux capteurs olfactifs 18 ; un prisme optique disposé contre une deuxième face, opposée à la première, de la couche métallique ; un dispositif d'éclairage à lumière collimatée et polarisée de cette deuxième face de la couche métallique via une face d'entrée de lumière du prisme optique ; et une caméra disposée en sortie de lumière du prisme optique pour la fourniture de la séquence S de signaux de mesure sous la forme d'une séquence d'images en niveaux de gris des sites réactifs sur lesquels sont disposés les capteurs olfactifs 18. Les sites réactifs de la première face de la couche métallique sont par exemple organisés en une grille matricielle de positionnement.

En variante, le transducteur 20 peut être un système d'amplification de variation d'indice optique par interférométrie de Mach-Zehnder, par exemple selon une technologie à matrice d'interféromètres de Mach-Zehnder, dite technologie MZI (de l'anglais « Mach-Zehnder Interferometer »), voire plus précisément une technologie MZI à interférences multimodales, dite technologie MZI/MMI (de l'anglais « Multi Mode Interference »). Un tel système est configuré pour mesurer tout changement d'un indice de réfraction dû à une interaction du fluide étudié avec au moins l'un quelconque des capteurs olfactifs grâce à un décalage de phases détectable entre un bras de référence de l'interféromètre et un bras de détection sur lequel ce site réactif quelconque est disposé. Le transducteur résultant fournit la séquence S de signaux de mesure qui se présente sous la forme d'une séquence d'images de décalages de phases, exprimés en Radian, des capteurs olfactifs 18.

Selon une autre variante, le transducteur 20 pourrait être un système d'amplification nano- ou micro-électromécanique NEMS (de l'anglais « Nano Electro-Mechanical System ») ou MEMS (de l'anglais « Micro Electro-Mechanical System »). Un tel système est configuré pour mesurer tout changement de fréquence de résonance d'une membrane vibrante sur laquelle est disposé l'un quelconque des capteurs olfactifs. Les sites réactifs sur lesquels sont placés les capteurs olfactifs 18 sont par exemple arrangés en une matrice de membranes vibrantes NEMS ou MEMS pour la fourniture de la séquence S de signaux de mesure qui se présente sous la forme d'une séquence de signaux de décalages de fréquences de résonance des capteurs olfactifs 18.

D'autres variantes sont envisageables par implémentation de tout autre dispositif de transduction physique (i.e. optique, mécanique, etc.) équivalent, en opérant une adaptation simple du dispositif électronique de caractérisation olfactive 10 qui ne sera pas décrite parce qu'à la portée de l'homme du métier. Quel que soit le choix du transducteur 20, l'idée générale reste de fonctionnaliser des sites réactifs à l'aide de capteurs olfactifs 18 (i.e. biocapteurs, polymères, nanotubes de carbones, etc.) de telle sorte qu'ils adsorbent et désorbent de manière différenciée les composés organiques volatils, de former une réponse d'interaction moléculaire différenciée des capteurs olfactifs, et d'amplifier la réponse sous la forme d'une séquence S de signaux électriques de mesure à l'aide d'un dispositif de transduction physique.

Le dispositif électronique de caractérisation olfactive 10 comporte en outre plusieurs modules fonctionnels qui vont être décrits ci-dessous. Dans l'exemple décrit, ces modules sont de nature logicielle. Ainsi, le dispositif 10 comporte un élément de type ordinateur 22 comportant une unité de traitement 24 et une zone de mémoire associée 26 dans laquelle plusieurs programmes d'ordinateurs ou plusieurs fonctions d'un même programme d'ordinateur sont enregistrés. Ces programmes d'ordinateurs comportent des instructions conçues pour être exécutées par l'unité de traitement 24 afin de réaliser les fonctions des modules logiciels. Ils sont présentés comme distincts, mais cette distinction est purement fonctionnelle. Ils pourraient tout aussi bien être regroupés selon toutes les combinaisons possibles en un ou plusieurs logiciels. Leurs fonctions pourraient aussi être au moins en partie micro programmées ou micro câblées dans des circuits intégrés dédiés, tels que des circuits numériques. Ainsi, en variante, l'ordinateur 22 pourrait être remplacé par un dispositif électronique composé uniquement de circuits numériques (sans programme d'ordinateur) pour la réalisation des mêmes fonctions.

Le dispositif électronique de caractérisation olfactive 10 comporte ainsi tout d'abord un module logiciel 28, destiné à être exécuté par l'unité de traitement 24, de commande du dispositif d'aspiration 14 (s'il est prévu dans de dispositif 10), de l'entrée d'air 16 (si elle est également prévue dans de dispositif 10) et du transducteur 20.

Il comporte en outre de façon optionnelle mais avantageuse un module logiciel 30, destiné à être exécuté par l'unité de traitement 24, de sélection, parmi les capteurs olfactifs 18 du dispositif électronique de caractérisation olfactive 10, d'un sous-ensemble de capteurs sensibles à des composants volatils caractéristiques d'une empreinte olfactive recherchée. Ces composants volatils caractéristiques peuvent varier d'une application ou d'un fluide étudié à l'autre de sorte que la sélection de capteurs olfactifs réalisée par le module logiciel 30 peut varier elle aussi et être paramétrée. Le sous-ensemble sélectionné comporte par exemple $M \geq 1$ capteur(s) olfactif(s), notamment avantageusement plusieurs capteurs olfactifs ($M \geq 2$).

Le dispositif électronique de caractérisation olfactive 10 comporte en outre un module logiciel 32, destiné à être exécuté par l'unité de traitement 24, pour extraire M sensorgrammes $SG_i$, $i \in \{1, ..., M\}$ respectivement représentatifs des interactions des M capteurs olfactifs sélectionnés avec les composés organiques volatils concernés à partir des valeurs propres à ces M capteurs olfactifs dans la séquence S de signaux de mesure fournie par le transducteur 20. Ces sensorgrammes $SG_i$, $i \in \{1, ..., M\}$ sont par exemple des signaux de réflectance exprimés en pourcentage selon un ratio de valeurs de luminance obtenues avec une lumière polarisée transversale sur des valeurs de luminance obtenues avec une même lumière polarisée à 90 degrés pour chacun des M capteurs olfactifs sélectionnés lorsque le transducteur 20 est un système d'imagerie de type SPR. Ils prennent la forme de signaux de décalages de phases et sont exprimés en Radian lorsque le transducteur est un système d'amplification MZI ou MZI/MMI. Ils prennent la forme de signaux de décalages de fréquences et sont exprimés en Hertz lorsque le transducteur est un système d'amplification NEMS ou MEMS.

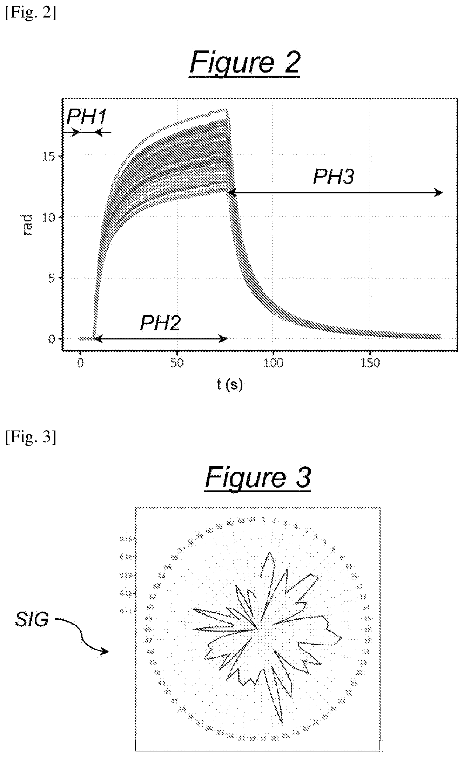

La figure 2 illustre ainsi les diagrammes temporels superposés d'une soixantaine de sensorgrammes $SG_i$, $i \in \{1, ..., M\}$ obtenus à l'aide d'un système d'amplification MZI ou MZI/MMI sur une durée d'environ 190 secondes selon un protocole de mesure bien maîtrisé, impliquant une commande du dispositif d'aspiration 14 et de l'entrée d'air 16, dans lequel :

- les capteurs olfactifs 18 sont tout d'abord exposés à un environnement fluidique de référence à fluide porteur sans présence des composés cibles d'un fluide à analyser au cours d'un premier état de référence identifiable par une première portion PH1 des sensorgrammes,

- ils sont ensuite exposés au fluide à analyser au cours d'un deuxième état analytique d'adsorption déclenché par une injection maîtrisée de ce fluide dans la chambre de mesure 12, ce deuxième état étant identifiable par une deuxième portion PH2 des sensorgrammes, et

- ils sont finalement exposés de nouveau à l'environnement fluidique de référence au cours d'un troisième état final de désorption par une évacuation maîtrisée du fluide à analyser hors de la chambre de mesure 12, ce troisième état étant identifiable par une troisième portion PH3 des sensorgrammes.

De retour à la figure 1, le dispositif électronique de caractérisation olfactive 10 comporte en outre un module logiciel 34 optionnel, destiné à être exécuté par l'unité de traitement 24, pour opérer un traitement préalable éventuel sur les M sensorgrammes $SG_i$, $i \in \{1, ..., M\}$ fournis par le module logiciel 32.

Ce traitement préalable comporte par exemple un filtrage passe-bas implémenté sous la forme d'un filtre numérique à réponse impulsionnelle finie ou infinie. Il s'agit de filtrer le bruit de mesure à hautes fréquences dans les signaux bruts tels que fournis par le module logiciel 32. Un filtre de Butterworth à réponse impulsionnelle finie du premier ordre et fréquence de coupure normalisée à 0,45 (i.e. valeur du ratio entre la fréquence de coupure et la fréquence d'échantillonnage égale à 0,45) convient.

Ce traitement préalable comporte par exemple en outre un calcul de norme au sens du document de brevet WO 2020/141281 A1 sur les M sensorgrammes $SG_i$, $i \in \{1, ..., M\}$ filtrés ou non pour l'obtention de M sensorgrammes filtrés et/ou normés $SG_i'$, $i \in \{1, ..., M\}$.

Le dispositif électronique de caractérisation olfactive 10 comporte en outre un module logiciel 36, destiné à être exécuté par l'unité de traitement 24, pour obtenir de façon bien connue et non détaillée une caractérisation ou signature SIG de la composition du fluide à analyser à partir des M sensorgrammes $SG_i$, $i \in \{1, ..., M\}$ ou $SG_i'$, $i \in \{1, ..., M\}$. Cette caractérisation ou signature SIG peut prendre la forme d'une signature olfactive normée telle qu'illustrée dans la figure 3 sous la forme d'un diagramme circulaire. On notera que ce module peut procéder en deux temps : tout d'abord l'obtention d'une première signature intermédiaire, puis la transformation de cette première signature intermédiaire par normalisation.

Le dispositif électronique de caractérisation olfactive 10 comporte en outre un module logiciel 38 optionnel, destiné à être exécuté par l'unité de traitement 24, pour transformer la signature SIG, normée ou non, en une autre signature YDEV simplifiée par réduction de composantes. Une méthode par analyse discriminante linéaire LDA (de l'anglais « Linear Discriminant Analysis »), analyse en composantes principales PCA (de l'anglais « Principal Component Analysis »), analyse en composantes indépendantes ICA (de l'anglais « Independent Component Analysis »), auto-encodeur, etc., convient. Pour une signature SIG à soixante-quatre composantes telle que celle de la figure 3, une signature YDEV simplifiée à deux ou trois composantes peut être obtenue.

Le dispositif électronique de caractérisation olfactive 10 comporte en outre une portion 40 de la zone de mémoire 26 pour le stockage d'un modèle paramétrique MOD, à au moins un paramètre réglable, de dérive par rapport à un dispositif électronique de caractérisation de référence. Ce modèle paramétrique de dérive est par exemple un modèle affine $MOD(\alpha, t)$ à deux paramètres réglables $\alpha$ et $t$, où $\alpha$ est un premier paramètre multiplicatif réglable du modèle affine et $t$ est un deuxième paramètre additif réglable du modèle affine. On notera que le dispositif électronique de caractérisation de référence peut être un dispositif électronique concrètement identifié comme étalon, un ensemble de dispositifs électroniques concrètement identifiés comme étalons, ou un dispositif électronique virtuel résultant d'un tel ensemble par exemple par moyenne, médiane ou tout autre calcul d'agrégation pertinent.

La portion 40 de la zone de mémoire 26 stocke en outre plusieurs signatures d'étalonnage de référence relatives respectivement à une interaction de plusieurs fluides d'étalonnage prédéterminés avec au moins un capteur du dispositif électronique de référence. Par souci de cohérence, le dispositif de référence comporte les mêmes capteurs olfactifs que le dispositif électronique de caractérisation olfactive 10. Conformément aux principes généraux de la présente invention, les fluides d'étalonnage prédéterminés sont répartis en plusieurs groupes d'étalonnage différents. Cela signifie que s'il y a un nombre quelconque $G \geq 2$ de groupes d'étalonnage différents alors il y a un nombre supérieur ou égal à $G$ de fluides d'étalonnage répartis dans ces $G$ groupes d'étalonnage à raison d'au moins un fluide d'étalonnage par groupe. La répartition peut se faire de façon aléatoire, par ressemblance de signatures selon un critère de ressemblance, ou toute autre règle de répartition à la portée de l'homme du métier. On notera par exemple $YREF_{r,i}$ la signature simplifiée d'étalonnage de référence du fluide d'étalonnage prédéterminé d'indice $i$ du groupe d'étalonnage d'indice $r$. Chaque signature $YREF_{r,i}$ peut par exemple être le résultat d'un calcul d'agrégation de valeurs de mesures prises sur plusieurs dispositifs électroniques de caractérisation de fluides, tel qu'une moyenne, une médiane, un maximum, un minimum ou autre. Le dispositif électronique de référence représente alors une agrégation théorique, i.e. virtuelle, de ces dispositifs électroniques de caractérisation de fluides.

Le dispositif électronique de caractérisation olfactive 10 comporte en outre un module logiciel 42, destiné à être exécuté par l'unité de traitement 24, pour réaliser un étalonnage par comparaison avec le dispositif électronique de caractérisation de référence. Ce module logiciel d'étalonnage 42 reçoit en entrée des signatures d'étalonnage obtenues par interaction des fluides d'étalonnage prédéterminés avec les capteurs olfactifs 18 du dispositif électronique de caractérisation olfactive 10 et les stocke dans une portion 44 de la zone de mémoire 26. Ces signatures d'étalonnage, propres au dispositif électronique de caractérisation olfactive 10, sont plus précisément obtenues par exécution des modules logiciels 28 à 38 sur chacun des fluides d'étalonnage prédéterminés. On notera par exemple $YDEV_{r,i}$ la signature simplifiée d'étalonnage obtenue du fluide d'étalonnage prédéterminé d'indice i du groupe d'étalonnage d'indice r.

Conformément aux principes généraux de la présente invention, l'étalonnage consiste à déterminer une valeur de chacun des paramètres réglables, en l'occurrence $\alpha$ et t pour le modèle affine précité, spécifiquement pour chaque groupe d'étalonnage. Plus précisément, il s'agit ici de déterminer chaque couple de valeurs spécifiques $(\alpha_r, t_r)$ pour chaque groupe d'étalonnage d'indice r, tel qu'il puisse être considéré que $YDEV_{r,i} = \alpha_r \cdot YREF_{r,i} + t_r$ pour chaque fluide d'étalonnage prédéterminé d'indice i de chaque groupe d'étalonnage d'indice r. Cette détermination est réalisée par le module logiciel 42, selon une méthode logicielle d'optimisation connue telle qu'une minimisation d'erreur quadratique ou une estimation du maximum de vraisemblance. Il stocke ensuite le résultat, c'est-à-dire les couples de valeurs spécifiques $(\alpha_r, t_r)$ des différents groupes d'étalonnage, dans la portion 44 de la zone de mémoire 26. Le dispositif électronique de caractérisation olfactive 10 peut alors être considéré comme étalonné.

On notera que les paramètres $\alpha_r$ et $t_r$ peuvent prendre des valeurs scalaires, mais également des valeurs vectorielles à autant de composantes que les signatures $YDEV_{r,i}$ et $YREF_{r,i}$.

On notera également que, conformément aux principes généraux de la présente invention, l'un des premier paramètre multiplicatif réglable $\alpha$ et deuxième paramètre additif réglable test indépendant des groupes d'étalonnage. Ainsi, le paramètre multiplicatif réglable $\alpha$ peut prendre des valeurs $\alpha_r$ différentes d'un groupe d'étalonnage à l'autre tandis que le paramètre additif réglable t reste constant à $t_0$, $t_0$ pouvant même être nul. De même, le paramètre additif réglable t peut prendre des valeurs $t_r$ différentes d'un groupe d'étalonnage à l'autre tandis que le paramètre multiplicatif réglable $\alpha$

reste constant à $\alpha_0$, $\alpha_0$ pouvant même être neutre à 1.

Le dispositif électronique de caractérisation olfactive 10 comporte en outre deux modules logiciels 46 et 48, destinés à être exécutés par l'unité de traitement 24, pour réaliser une correction sur la signature mesurée d'un fluide quelconque à caractériser. Le module logiciel 46 réalise plus précisément la sélection d'un groupe d'étalonnage à associer au fluide à caractériser en fonction de sa signature, tandis que le module logiciel 48 corrige cette signature à l'aide des valeurs spécifiques des paramètres réglables du groupe sélectionné.

Concrètement, le module logiciel de sélection 46 reçoit une signature en entrée, par exemple une signature simplifiée YDEV, obtenue par interaction du fluide à analyser avec les capteurs olfactifs 18 du dispositif électronique de caractérisation olfactive 10. Cette signature est plus précisément obtenue par exécution des modules logiciels 28 à 38 sur le fluide à caractériser. Le module logiciel de sélection 46 compare alors cette signature simplifiée YDEV avec les signatures d'étalonnage $YDEV_{r,i}$ et sélectionne le groupe d'étalonnage, d'indice r̂, qui semble le plus représentatif de cette signature selon un critère prédéterminé. Ceci peut être réalisé selon de nombreuses méthodes bien connues, plus ou moins simples. Avantageusement, la sélection est réalisée par classification automatique de la signature simplifiée YDEV dans l'un des groupes d'étalonnage par comparaison de cette signature avec la ou les signature(s) d'étalonnage obtenue(s) du ou des fluide(s) d'étalonnage de chaque groupe d'étalonnage. Une méthode des k plus proches voisins $(k \geq 1)$ est en particulier tout à fait adaptée à la situation.

Le module logiciel de correction 48 reçoit l'indice r̂ du groupe sélectionné en entrée et applique les valeurs de paramètres correspondantes $\alpha_{r̂}$ et $t_{r̂}$ à une inversion du modèle paramétrique de dérive. Concrètement, dans l'exemple du modèle affine précité, cela revient à appliquer la correction suivante sur la signature YDEV :

$$YCOR = \frac{(YDEV - t_{r̂})}{\alpha_{r̂}}.$$

YCOR est donc la signature simplifiée du fluide à caractériser après correction.

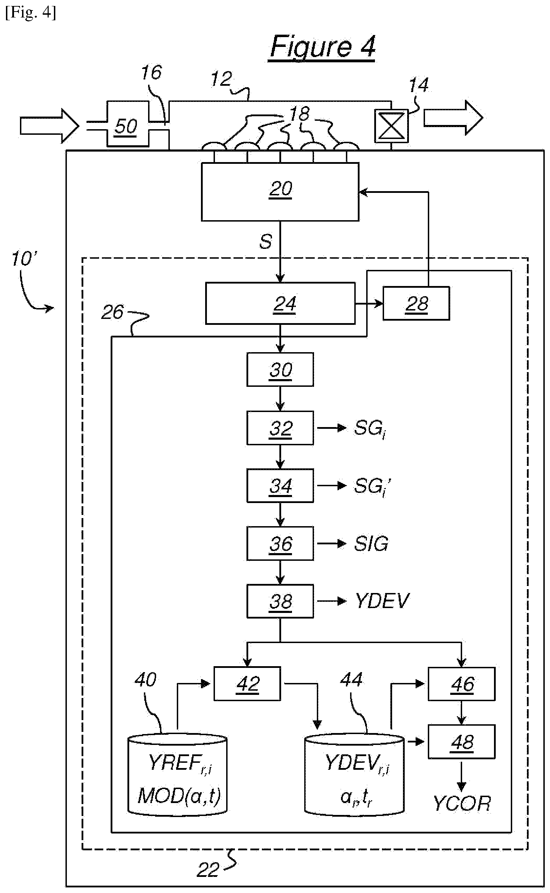

Selon un deuxième mode de réalisation de la présente invention, le dispositif électronique 10' de caractérisation olfactive de fluide représenté schématiquement sur la figure 4 diffère de celui de la figure 1 en ce que l'entrée d'air 16 est raccordée à un concentrateur thermodésorbeur 50 par l'intermédiaire duquel elle reçoit le fluide à analyser. Tous les autres éléments constitutifs du dispositif électronique 10' restent identiques à ceux du dispositif électronique 10 de sortent qu'ils conservent les mêmes références. Le concentrateur thermodésorbeur 50 est un appareil connu qui ne sera pas détaillé. Son fonctionnement consiste à accumuler les composés d'un fluide par adsorption sur une résine. Celle-ci est ensuite chauffée à une certaine température de consigne, par exemple 200°C, pour déclencher la désorption de ces composés de la résine. Une fois la température de consigne atteinte, les composés sont injectés dans le dispositif électronique 10'.

Il en résulte l'injection rapide d'un pic de concentration plutôt que l'injection en continu des composés dans le contexte d'une mesure telle qu'elle a été décrite dans le mode de réalisation précédent. Ce pic de concentration engendre alors des signaux temporels distincts eux aussi en forme de pics, plutôt qu'avec la forme temporellement étendue des sensorgrammes de la figure 2. Mais par un abus de langage tout à fait légitime, ces signaux temporels distincts peuvent eux aussi être appelés sensorgrammes puisqu'ils restent malgré tout une réponse du transducteur 20 à l'introduction d'un fluide à analyser dans la chambre de mesure 12, même si cette introduction se fait différemment.

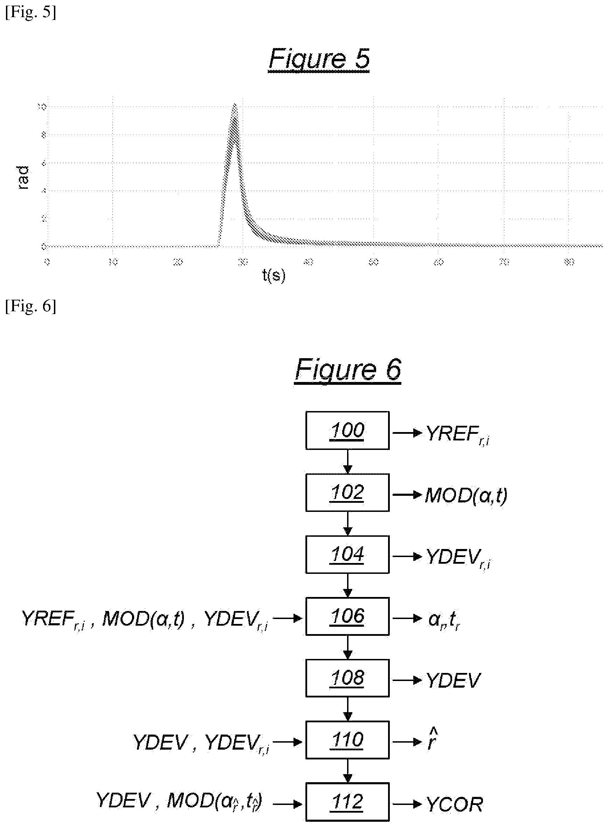

La figure 5 illustre ainsi les diagrammes temporels superposés d'une soixantaine de sensorgrammes $SG_i$, $i \in \{1, ..., M\}$ obtenus à l'aide d'un système d'amplification MZI ou MZI/MMI sur une durée d'environ 80 secondes selon un protocole de mesure bien maîtrisé à l'aide du concentrateur thermodésorbeur 50.

Les étapes successives d'un procédé d'étalonnage du dispositif électronique de caractérisation olfactive 10 ou 10' vont maintenant être détaillées en référence à la figure 6.

Au cours d'une étape 100, les signatures de référence $YREF_{r,i}$ des produits d'étalonnage prédéterminés sont stockées en mémoire 40 du dispositif électronique de caractérisation olfactive 10 ou 10'. Leur acquisition se fait par exécution de modules logiciels, équivalents aux modules logiciels 28 à 38, d'un dispositif électronique de référence, virtuel ou non, similaire au dispositif électronique de caractérisation olfactive 10 ou 10'.

Au cours d'une étape 102 réalisée avant, pendant ou après l'étape 100, le modèle paramétrique MOD($\alpha$,t) de dérive du dispositif électronique de caractérisation olfactive 10 ou 10' à étalonner par rapport au dispositif électronique de référence est également stocké en mémoire 40.

Au cours d'une étape 104 réalisée par exécution des modules logiciels 28 à 38, les signatures d'étalonnage $YDEV_{r,i}$ des produits d'étalonnage prédéterminés sont obtenues par interaction avec les capteurs 18 du dispositif électronique de caractérisation olfactive 10 ou 10'.

Au cours d'une étape 106 réalisée par exécution du module logiciel 42, les signatures d'étalonnage $YDEV_{r,i}$ sont stockées en mémoire 44. Au cours de cette même étape, les valeurs spécifiques ($\alpha_r$,$t_r$) des paramètres réglables du modèle paramétrique MOD($\alpha$,t) sont déterminées et enregistrées en mémoire 44 pour chacun des différents groupes d'étalonnage.

A l'issue de cette étape, le dispositif électronique de caractérisation olfactive 10 ou 10' peut être considéré comme étalonné.

Au cours d'une étape suivante 108, réalisée par exécution des modules logiciels 28 à 38, un fluide à caractériser est approché du dispositif électronique de caractérisation olfactive 10 ou 10' pour une interaction avec ses capteurs olfactifs 18. Il en résulte la signature simplifiée YDEV.

Au cours d'une étape suivante 110, réalisée par exécution du module logiciel 46, le groupe d'étalonnage, d'indice r̂, considéré comme le plus représentatif de la la signature simplifiée YDEV est sélectionné.

Enfin, au cours d'une dernière étape 112, réalisée par exécution du module logiciel 48, cette signature YDEV est corrigée en $YCOR = \dfrac{(YDEV - t_{\hat{r}})}{\alpha_{\hat{r}}}$ par inversion du modèle paramétrique de dérive.

On notera que le procédé d'étalonnage précité prévoit une éventuelle simplification des signatures par réduction de dimension avant application des paramètres d'étalonnage, de sorte que ceux-ci sont alors adaptés à la dimension réduite après simplification lorsqu'ils sont vectoriels. Mais en variante, il est tout à fait possible d'opérer l'étalonnage avant la réduction de dimension, de sorte que les paramètres d'étalonnage sont alors adaptés à la dimension initiale avant simplification lorsqu'ils sont vectoriels.

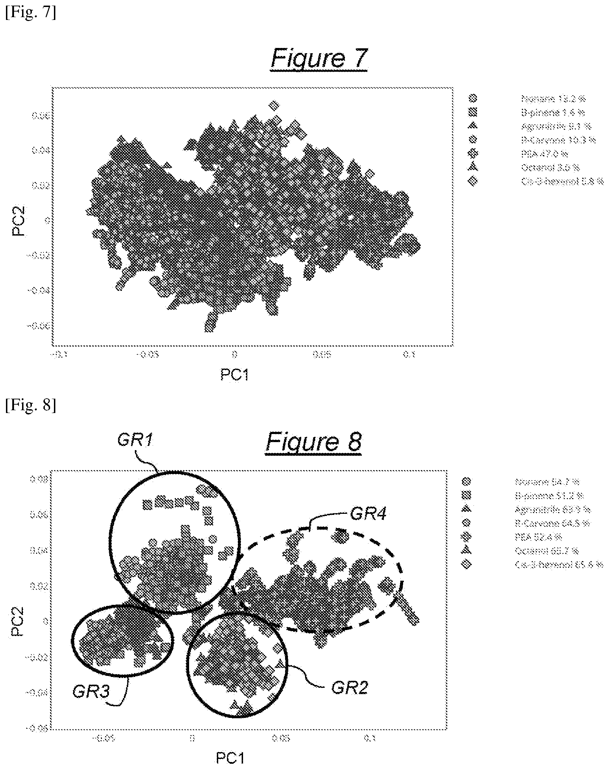

Les diagrammes des figures 7 et 8 illustrent des résultats expérimentaux obtenus sur des échantillons fluidiques prédéterminés dont les signatures sont mesurées sur un grand nombre de dispositifs électroniques de caractérisation différents. Les signatures sont simplifiées par analyse en composantes principales et deux premières composantes principales PC1, PC2 sont retenues pour caractériser les échantillons. Sept types d'échantillons sont proposés à la mesure : des échantillons de nonane, des échantillons de β-pinène, des échantillons d'agrunitrile, des échantillons de R-carvone, des échantillons de PEA (alcool phényléthylique), des échantillons d'octanol et des échantillons de cis-3-hexenol.

La figure 7 illustre la distribution de différentes signatures simplifiées obtenues, sans aucun étalonnage préalable, dans le plan des composantes principales PC1 et PC2. Un score CQS (de l'anglais « Clustering Quality Score ») tel que par exemple enseigné dans le document de brevet WO 2022/053690 A1 peut être calculé pour chacun des types d'échantillons proposés à la mesure et moyenné sur l'ensemble des échantillons. On voit nettement qu'il est difficile de caractériser les différents types d'échantillons sur la figure 7. D'ailleurs les échantillons de nonane présentent expérimentalement un score CQS de 13,2 %, ceux de β-pinène un score CQS de 1,6 %, ceux d'agrunitrile un score CQS de 9,1 %, ceux de R-carvone un score CQS de 10,3 %, ceux de PEA un score CQS de 47 %, ceux d'octanol un score CQS de 3,0 % et ceux de cis-3-hexenol un score CQS de 5,8 %, pour un score CQS moyen global de 12,9 %. Ce n'est pas très satisfaisant.

Lorsqu'un étalonnage selon la présente invention est réalisé, un résultat tel que celui illustré sur le diagramme de la figure 8 peut être obtenu. Trois groupes d'étalonnage sont par exemple définis : un premier groupe composé des échantillons de nonane et de β-pinène, un deuxième groupe d'octanol et de cis-3-hexenol et un troisième groupe d'agrunitrile et de R-carvone. Les échantillons de PEA ne sont par exemple pas exploités pour l'étalonnage. Le modèle de dérive paramétrique retenu est un modèle affine dans lequel les valeurs $\alpha_r$ du paramètre multiplicatif $\alpha$ sont scalaires et les valeurs $t_r$ du paramètre additif t sont vectorielles. La figure 8 illustre ainsi la distribution de différentes signatures simplifiées et corrigées obtenues, après l'étalonnage précité, dans le plan des composantes principales PC1 et PC2. Plus précisément, la figure 8 illustre un cas de simplification des signatures après étalonnage, de sorte que les valeurs vectorielles $t_r$ du paramètre additif t sont de la dimension des signatures avant réduction de dimension. On voit que quatre groupes de signatures se détachent plus distinctement que dans la figure 7. Un premier groupe GR1 rassemble l'essentiel des échantillons de nonane et de β-pinène. Un deuxième groupe GR2 rassemble l'essentiel des échantillons d'octanol et de cis-3-hexenol. Un troisième groupe GR3 rassemble l'essentiel des échantillons d'agrunitrile et de R-carvone. Un quatrième groupe GR4, représenté en pointillés parce que relatif à des composés qui n'ont pas fait partie d'un groupe d'étalonnage, rassemble l'essentiel des échantillons de PEA. En toute logique, les échantillons de nonane présentent expérimentalement un score CQS amélioré de 54,7 %, ceux de β-pinène un score CQS amélioré de 51,2 %, ceux d'agrunitrile un score CQS amélioré de 63,9 %, ceux de R-carvone un score CQS amélioré de 64,5 %, ceux de PEA un score CQS amélioré de 52,4 %, ceux d'octanol un score CQS amélioré de 65,7 % et ceux de cis-3-hexenol un score CQS amélioré de 65,6 %, pour un score CQS moyen global nettement amélioré de 59,7 %.

Il apparaît clairement qu'un précédé d'étalonnage tel que celui qui vient d'être détaillé permet de compenser efficacement les dérives, dans le temps et d'un dispositif à l'autre, de dispositifs électroniques de caractérisation de fluides. Une discrimination de fluides à caractériser est ainsi améliorée.

On notera par ailleurs que l'invention n'est pas limitée aux modes de réalisation décrits précédemment.

En particulier, un seul modèle paramétrique de dérive a été présenté mais d'autres modèles peuvent être envisagés en fonction de la réalité des dérives possibles. Il convient juste de noter que plus le nombre de paramètres à régler est important, plus le nombre de groupes d'étalonnage ou le nombre de fluides d'étalonnage par groupe doit également être important.

Il apparaîtra plus généralement à l'homme de l'art que diverses modifications peuvent être apportées aux modes de réalisation décrits ci-dessus, à la lumière de l'enseignement qui vient de lui être divulgué. Dans la présentation détaillée de l'invention qui est faite précédemment, les termes utilisés ne doivent pas être interprétés comme limitant l'invention aux modes de réalisation exposés dans la présente description, mais doivent être interprétés pour y inclure tous les équivalents dont la prévision est à la portée de l'homme de l'art en appliquant ses connaissances générales à la mise en œuvre de l'enseignement qui vient de lui être divulgué.

## Revendications

1. Procédé d'étalonnage d'un dispositif électronique (10) de caractérisation d'un fluide à au moins un capteur (18) conçu pour interagir avec le fluide, par comparaison avec un dispositif électronique de caractérisation de référence, comportant les étapes suivantes :

    - stockage (100) en mémoire (40) d'une pluralité de signatures d'étalonnage de référence ($YREF_{r,i}$) relatives respectivement à une interaction d'une pluralité de fluides d'étalonnage prédéterminés avec au moins un capteur du dispositif électronique de référence ;
    - stockage (102) en mémoire (40) d'un modèle paramétrique ($MOD(\alpha,t)$), à au moins un paramètre réglable ($\alpha,t$), de dérive du dispositif électronique (10) à étalonner par rapport au dispositif électronique de référence ;
    - pour chacun de la pluralité de fluides d'étalonnage prédéterminés, obtention (104) d'une signature d'étalonnage ($YDEV_{r,i}$) par interaction avec ledit au moins un capteur (18) du dispositif électronique (10) à étalonner ;
    - détermination (106) d'au moins une valeur ($\alpha_r$, $t_r$) dudit au moins un paramètre réglable ($\alpha,t$) par comparaison des signatures d'étalonnage obtenues ($YDEV_{r,i}$) avec les signatures d'étalonnage de référence ($YREF_{r,i}$) ;

    **caractérisé en ce que** les fluides d'étalonnage prédéterminés sont répartis en plusieurs groupes d'étalonnage différents et la détermination (106) de ladite au moins une valeur ($\alpha_r,t_r$) dudit au moins un paramètre réglable ($\alpha,t$) est réalisée spécifiquement pour chaque groupe d'étalonnage.

2. Procédé d'étalonnage selon la revendication 1, comportant en outre une étape (110, 112) d'application de ladite au moins une valeur ($\alpha_r,t_r$) de paramètre réglable dans le modèle paramétrique de dérive ($MOD(\alpha,t)$) pour corriger toute signature ($YDEV$) obtenue par interaction d'un fluide quelconque avec ledit au moins un capteur (18) du dispositif électronique (10) à étalonner, cette étape comportant la sélection (110) d'un groupe d'étalonnage ($\hat{r}$) à associer au fluide quelconque en fonction de sa signature ($YDEV$), puis la correction (112) de sa signature à l'aide de ladite au moins une valeur de paramètre réglable du groupe sélectionné.

3. Procédé d'étalonnage selon la revendication 2, dans lequel la sélection (110) d'un groupe d'étalonnage à associer au fluide quelconque comporte une classification automatique de sa signature (YDEV) dans l'un des groupes d'étalonnage par comparaison de cette signature avec la ou les signature(s) d'étalonnage obtenue(s) du ou des fluide(s) d'étalonnage de chaque groupe d'étalonnage.

4. Procédé d'étalonnage selon la revendication 2 ou 3, dans lequel la correction (112) de la signature (YDEV) du fluide quelconque comporte une inversion du modèle paramétrique de dérive $(MOD(\alpha,t))$ dans lequel est appliquée ladite au moins une valeur de paramètre réglable du groupe d'étalonnage sélectionné.

5. Procédé d'étalonnage selon l'une quelconque des revendications 1 à 4, dans lequel l'obtention (104) d'une signature d'étalonnage $(YDEV_{r,i})$ pour chacun de la pluralité de fluides d'étalonnage prédéterminés comporte l'obtention d'une première signature d'étalonnage à N composante(s), $N \geq 1$, puis la transformation de cette première signature d'étalonnage par normalisation et/ou réduction de composantes.

6. Procédé d'étalonnage selon l'une quelconque des revendications 1 à 5, dans lequel le modèle paramétrique de dérive $(MOD(\alpha,t))$ est un modèle affine à deux paramètres réglables, $YDEV_{r,i} = \alpha_r \cdot YREF_{r,i} + t_r$, où $YDEV_{r,i}$ est la signature d'étalonnage obtenue du fluide d'étalonnage prédéterminé d'indice i du groupe d'étalonnage d'indice r, $YREF_{r,i}$ est la signature d'étalonnage de référence du fluide d'étalonnage prédéterminé d'indice i du groupe d'étalonnage d'indice r, $\alpha_r$ est la valeur d'un premier paramètre multiplicatif réglable du modèle affine pour le groupe d'étalonnage d'indice r et $t_r$ est la valeur d'un deuxième paramètre additif réglable du modèle affine pour le groupe d'étalonnage d'indice r.

7. Procédé d'étalonnage selon les revendications 2 et 6, dans lequel la correction (112) de la signature (YDEV) du fluide quelconque comporte le calcul $YCOR = (YDEV - t_{\hat{r}})/\alpha_{\hat{r}}$, où YDEV est la signature obtenue par interaction du fluide quelconque avec ledit au moins un capteur (18) du dispositif électronique (10) à étalonner, YCOR est la signature du fluide quelconque après correction et $\hat{r}$ est l'indice du groupe d'étalonnage sélectionné.

8. Procédé d'étalonnage selon la revendication 6 ou 7, dans lequel l'un des premier paramètre multiplicatif réglable et deuxième paramètre additif réglable est indépendant des groupes d'étalonnage.

9. Programme d'ordinateur téléchargeable depuis un réseau de communication et/ou enregistré sur un support lisible par ordinateur (22) comprenant des instructions qui, lorsqu'il est exécuté par une unité de traitement (24) d'un dispositif électronique de caractérisation de fluide (10) à étalonner, cette unité de traitement (24) étant apte à échanger des données avec une mémoire (40) dans laquelle sont stockés :

  - une pluralité de signatures d'étalonnage de référence $(YREF_{r,i})$ relatives respectivement à une interaction d'une pluralité de fluides d'étalonnage prédéterminés avec au moins un capteur d'un dispositif électronique de caractérisation de référence ;
  - un modèle paramétrique $(MOD(\alpha,t))$, à au moins un paramètre réglable, de dérive du dispositif électronique de caractérisation de fluide (10) à étalonner par rapport au dispositif électronique de référence ;

  conduisent l'unité de traitement (24) à exécuter les étapes suivantes en interaction avec la mémoire (40) et avec au moins un capteur (18) du dispositif électronique de caractérisation de fluide (10) à étalonner :

    - pour chacun de la pluralité de fluides d'étalonnage prédéterminés, obtention (104) d'une signature d'étalonnage $(YDEV_{r,i})$ par interaction avec ledit au moins un capteur (18) ;
    - détermination (106) d'au moins une valeur $(\alpha_r,t_r)$ dudit au moins un paramètre réglable $(\alpha,t)$ par comparaison des signatures d'étalonnage obtenues $(YDEV_{r,i})$ avec les signatures d'étalonnage de référence $(YREF_{r,i})$ ;

    **caractérisé en ce que**, les fluides d'étalonnage prédéterminés étant répartis en plusieurs groupes d'étalonnage différents, les instructions sont plus précisément conçues pour que la détermination (106) de ladite au moins une valeur $(\alpha_r,t_r)$ dudit au moins un paramètre réglable $(\alpha,t)$ soit réalisée spécifiquement pour chaque groupe d'étalonnage.

10. Dispositif électronique étalonné (10) de caractérisation d'un fluide par la fourniture d'une signature (YDEV) obtenue à partir d'un signal électrique (S) de mesure, comportant :

    - au moins un capteur (18) conçu pour interagir avec le fluide ;
    - un transducteur (20) conçu pour fournir, en interaction avec ledit au moins un capteur (18), le signal électrique (S) de mesure ;
    - une mémoire (40, 44) de stockage :

• d'un modèle paramétrique (MOD($\alpha$,t)), à au moins un paramètre réglable ($\alpha$,t), de dérive du dispositif électronique étalonné (10) par rapport à un dispositif électronique de caractérisation de référence,

• d'au moins une valeur ($\alpha_r$,$t_r$) dudit au moins un paramètre réglable ($\alpha$,t), calculée par un étalonnage préalable du dispositif électronique étalonné par comparaison avec le dispositif électronique de référence sur la base d'une pluralité de fluides d'étalonnage prédéterminés ; et

- un processeur (24) de correction de la signature (YDEV) par application de ladite au moins une valeur de paramètre réglable ;

**caractérisé en ce que** :

- les fluides d'étalonnage prédéterminés sont répartis en plusieurs groupes d'étalonnage différents et la mémoire stocke au moins une valeur spécifique ($\alpha_r$,$t_r$) dudit au moins un paramètre réglable ($\alpha$,t) pour chacun des groupes d'étalonnage ; et
- le processeur (24) est plus précisément programmé (46, 48) pour sélectionner un groupe d'étalonnage à associer au fluide à caractériser en fonction de sa signature (YDEV), puis corriger sa signature à l'aide de ladite au moins une valeur spécifique ($\alpha_r$,$t_r$) de paramètre réglable du groupe sélectionné.

**Patentansprüche**

1. Verfahren zum Kalibrieren einer elektronischen Vorrichtung (10) zum Charakterisieren eines Fluids an mindestens einem Sensor (18), der gestaltet ist, um mit dem Fluid zu interagieren, durch Vergleich mit einer elektronischen Referenzcharakterisierungsvorrichtung, die folgenden Schritte beinhaltend:

   - Speichern (100), in einem Speicher (40), einer Vielzahl von Referenzkalibriersignaturen (YREF$_{r,i}$) jeweils in Bezug auf eine Interaktion einer Vielzahl von vorbestimmten Kalibrierfluiden mit mindestens einem Sensor der elektronischen Referenzvorrichtung;
   - Speichern (102), in einem Speicher (40), eines parametrischen Modells (MOD($\alpha$,t)) mit mindestens einem einstellbaren Parameter ($\alpha$,t) eines Abdriftens der elektronischen Vorrichtung (10), die in Bezug auf die elektronische Referenzvorrichtung zu kalibrieren ist;
   - für jedes der Vielzahl von vorbestimmten Kalibrierfluiden, Erhalten (104) einer Kalibriersignatur (YDEV$_{r,i}$) durch Interaktion mit dem mindestens einen Sensor (18) der zu kalibrierenden elektronischen Vorrichtung (10) ;
   - Bestimmen (106) mindestens eines Werts ($\alpha_r$,$t_r$) des mindestens einen einstellbaren Parameters ($\alpha$,t) durch Vergleich der erhaltenen Kalibriersignaturen (YDEV$_{r,i}$) mit den Referenzkalibriersignaturen (YREF$_{r,i}$) ;

**dadurch gekennzeichnet, dass** die vorbestimmten Kalibrierfluide in mehrere verschiedene Kalibriergruppen aufgeteilt sind und das Bestimmen (106) des mindestens einen Werts ($\alpha_r$,$t_r$) des mindestens einen einstellbaren Parameters ($\alpha$,t) spezifisch für jede Kalibriergruppe durchgeführt wird.

2. Kalibrierverfahren nach Anspruch 1, weiter einen Schritt (110, 112) zum Anwenden des mindestens einen einstellbaren Parameterwerts ($\alpha_r$,$t_r$) im Abdriftparametriermodell (MOD($\alpha$,t)) umfassend, um jede Signatur (YDEV), die durch Interaktion eines beliebigen Fluids mit dem mindestens einen Sensor (18) der zu kalibrierenden elektronischen Vorrichtung (10) erhalten wird, zu korrigieren, wobei dieser Schritt das Auswählen (110) einer Kalibriergruppe (r̂) beinhaltet, die dem beliebigen Fluid in Abhängigkeit von seiner Signatur (YDEV) zuzuordnen ist, und dann das Korrigieren (112) seiner Signatur mithilfe des mindestens einen einstellbaren Parameterwerts der ausgewählten Gruppe.

3. Kalibrierverfahren nach Anspruch 2, wobei das Auswählen (110) einer Kalibriergruppe, die einem beliebigen Fluid zuzuordnen ist, eine automatische Klassifizierung ihrer Signatur (YDEV) in einer der Kalibriergruppen durch Vergleichen dieser Signatur mit der/den von dem/den Kalibrierfluid(en) jeder Kalibriergruppe erhaltenen Kalibriersignatur(en) umfasst.

4. Kalibrierverfahren nach Anspruch 2 oder 3, wobei das Korrigieren (112) der Signatur (YDEV) des beliebigen Fluids eine Invertierung des parametrischen Abdriftmodells (MOD($\alpha$,t)) beinhaltet, bei dem der mindestens eine einstellbare Parameterwert der ausgewählten Kalibriergruppe angewendet wird.

5. Kalibrierverfahren nach einem der Ansprüche 1 bis 4, wobei das Erhalten (104) einer Kalibriersignatur (YDEV$_{r,i}$) für jede der Vielzahl von vorbestimmten Kalibrierfluiden das Erhalten einer ersten Kalibriersignatur mit N Komponente(n), wobei N $\geq$ 1, und anschließend das Transformieren dieser ersten Kalibriersignatur durch Normieren und/oder Reduzieren von Komponenten beinhaltet.

6. Kalibrierverfahren nach einem der Ansprüche 1 bis 5, wobei das parametrische Abdriftmodell (MOD($\alpha$,t)) ein affines Modell mit zwei einstellbaren Parametern, YDEV$_{r,i}$ =$\alpha_r$·YREF$_{r,i}$+$t_r$, ist, wobei YDEV$_{r,i}$

die Kalibriersignatur ist, die aus dem vorbestimmten Kalibrierfluid mit dem Index i der Kalibriergruppe mit dem Index r erhalten wird, $YREF_{r,i}$ die Referenzkalibriersignatur des vorbestimmten Kalibrierfluids mit dem Index i der Kalibriergruppe mit dem Index r ist, $\alpha_r$ der Wert eines ersten einstellbaren multiplikativen Parameters des affinen Modells für die Kalibriergruppe mit dem Index r ist und $t_r$ der Wert eines zweiten einstellbaren additiven Parameters des affinen Modells für die Kalibriergruppe mit dem Index r ist.

7. Kalibrierverfahren nach den Ansprüchen 2 und 6, wobei das Korrigieren (112) der Signatur (YDEV) des beliebigen Fluids das Berechnen $YCOR=(YDEV-t_{\hat{f}}) / \alpha_{\hat{f}}$ umfasst, wobei YDEV die Signatur ist, die durch Interaktion des beliebigen Fluids mit dem mindestens einen Sensor (18) der zu kalibrierenden elektronischen Vorrichtung (10) erhalten wird, YCOR die Signatur des beliebigen Fluids nach der Korrektur ist und $\hat{f}$ der Index der ausgewählten Kalibriergruppe ist.

8. Kalibrierverfahren nach Anspruch 6 oder 7, wobei der eine der ersten einstellbaren multiplikativen Parameter und der zweiten einstellbaren additiven Parameter unabhängig von den Kalibriergruppen ist.

9. Computerprogramm, das von einem Kommunikationsnetzwerk heruntergeladen und/oder auf einem computerlesbaren Medium (22) gespeichert werden kann, das Anweisungen umfasst, die, wenn es von einer Verarbeitungseinheit (24) einer zu kalibrierenden elektronischen Vorrichtung (10) zum Charakterisieren eines Fluids ausgeführt wird, diese Verarbeitungseinheit (24) imstande ist, Daten mit einem Speicher (40) auszutauschen, in dem gespeichert sind :

   - eine Vielzahl von Referenzkalibriersignaturen $(YREF_{r,i})$, jeweils in Bezug auf eine Interaktion einer Vielzahl von vorbestimmten Kalibrierfluiden mit mindestens einem Sensor einer elektronischen Referenzcharakterisierungsvorrichtung;
   - ein parametrisches Modell $(MOD(\alpha,t))$ mit mindestens einem einstellbaren Abdriftparameter der elektronischen Vorrichtung (10) zum Charakterisieren eines Fluids, die in Bezug auf die elektronische Referenzvorrichtung zu kalibrieren ist;
   die Verarbeitungseinheit (24) veranlassen, die folgenden Schritte in Interaktion mit dem Speicher (40) und mit mindestens einem Sensor (18) der zu kalibrierenden elektronischen Vorrichtung zum Charakterisieren eines Fluids (10) auszuführen:

   - für jedes der Vielzahl von vorbestimmten Kalibrierfluiden, Erhalten (104) einer Kalibriersignatur $(YDEV_{r,i})$ durch Interaktion mit dem mindestens einen Sensor (18) ;
   - Bestimmen (106) mindestens eines Werts $(\alpha_r ,t_r)$ des mindestens einen einstellbaren Parameters $(\alpha,t)$ durch Vergleich der erhaltenen Kalibriersignaturen $(YDEV_{r,i})$ mit den Referenzkalibriersignaturen $(YREF_{r,i})$ ;

   **dadurch gekennzeichnet, dass** die vorbestimmten Kalibrierfluide in mehrere verschiedene Kalibriergruppen aufgeteilt sind und die Anweisungen genauer gestaltet sind, damit das Bestimmen (106) des mindestens einen Werts $(\alpha_r ,t_r)$ des mindestens einstellbaren Parameters $(\alpha,t)$ spezifisch für jede Kalibriergruppe durchgeführt wird.

10. Kalibrierte elektronische Vorrichtung (10) zum Charakterisieren eines Fluids durch Bereitstellen einer Signatur (YDEV), die aus einem elektrischen Messsignal (S) erhalten wird, umfassend:

   - mindestens einen Sensor (18), der gestaltet ist, um mit dem Fluid zu interagieren;
   - einen Wandler (20), der gestaltet ist, um in Interaktion mit dem mindestens einen Sensor (18) das elektrische Messsignal (S) bereitzustellen ;
   - einen Speicher (40, 44) zum Speichern:

   • eines parametrischen Modells $(MOD(\alpha,t))$ mit mindestens einem einstellbaren Parameter $(\alpha,t)$ eines Abdriftens der kalibrierten elektronischen Vorrichtung (10) in Bezug auf eine elektronische Referenzcharakterisierungsvorrichtung,
   • mindestens eines Werts $(\alpha_r ,t_r)$ des mindestens einen einstellbaren Parameters $(\alpha,t)$, der durch eine vorherige Kalibrierung der kalibrierten elektronischen Vorrichtung durch Vergleich mit der elektronischen Referenzvorrichtung basierend auf einer Vielzahl von vorbestimmten Kalibrierfluiden berechnet wird ; und

   - einen Prozessor (24) zum Korrigieren der Signatur (YDEV) durch Anwenden des mindestens einen einstellbaren Parameterwerts;

   **dadurch gekennzeichnet, dass**:

   - die vorbestimmten Kalibrierfluide in mehrere verschiedene Kalibriergruppen aufgeteilt sind und der Speicher mindestens einen spezifischen Wert $(\alpha_r ,t_r)$ des mindestens einen einstellbaren Parameters $(\alpha,t)$ für jede der Kalibriergruppen speichert; und

- der Prozessor (24) genauer programmiert (46, 48) ist, um eine Kalibriergruppe auszuwählen, die dem zu charakterisierenden Fluid in Abhängigkeit von seiner Signatur (YDEV) zuzuordnen ist, und dann seine Signatur mithilfe des mindestens einen spezifischen Werts $(\alpha_r, t_r)$ eines einstellbaren Parameters der ausgewählten Gruppe zu korrigieren.

**Claims**

1. A method for calibrating an electronic device (10) for characterizing a fluid with at least one sensor (18) designed to interact with the fluid, by comparison with a reference electronic characterization device, comprising the following steps:

   - storing (100), in memory (40), a plurality of reference calibration signatures ($YREF_{r,i}$) relating respectively to an interaction of a plurality of predetermined calibration fluids with at least one sensor of the reference electronic device;
   - storing (102), in memory (40), a parametric model ($MOD(\alpha,t)$), with at least one adjustable parameter $(\alpha,t)$, of a drift of the electronic device (10) to be calibrated with respect to the reference electronic device;
   - for each of the plurality of predetermined calibration fluids, obtaining (104) a calibration signature ($YDEV_{r,i}$) by interaction with said at least one sensor (18) of the electronic device (10) to be calibrated;
   - determining (106) at least one value $(\alpha_r, t_r)$ of said at least one adjustable parameter $(\alpha,t)$ by comparison of the obtained calibration signatures ($YDEV_{r,i}$) with the reference calibration signatures ($YREF_{r,i}$);

   **characterized in that** the predetermined calibration fluids are divided into a plurality of different calibration groups and the determination (106) of said at least one value $(\alpha_r, t_r)$ of said at least one adjustable parameter $(\alpha,t)$ is carried out specifically for each calibration group.

2. The calibration method according to claim 1, further comprising a step (110, 112) for applying said at least one adjustable parameter value $(\alpha_r, t_r)$ in the parametric drift model ($MOD(\alpha,t)$) to correct any signature (YDEV) obtained by interaction of any fluid with said at least one sensor (18) of the electronic device (10) to be calibrated, this step comprising the selection (110) of a calibration group (r̂) to be associated with said any fluid based on its signature (YDEV), then the correction (112) of its signature using said at least one adjustable parameter value of the selected group.

3. The calibration method according to claim 2, wherein the selection (110) of a calibration group to be associated with said any fluid involves automatic classification of its signature (YDEV) in one of the calibration groups by comparison of this signature with the calibration signature(s) obtained from the calibration fluid(s) in each calibration group.

4. The calibration method according to claim 2 or 3, wherein the correction (112) of said any fluid signature (YDEV) comprises an inversion of the parametric drift model ($MOD(\alpha,t)$) in which said at least one adjustable parameter value of the selected calibration group is applied.

5. The calibration method according to any one of claims 1 to 4, wherein obtaining (104) a calibration signature ($YDEV_{r,i}$) for each of the plurality of predetermined calibration fluids comprises obtaining a first calibration signature with N component(s), $N \geq 1$, then transforming this first calibration signature by normalization and/or component reduction.

6. The calibration method according to any one of claims 1 to 5, wherein the parametric drift model ($MOD(\alpha,t)$) is an adjustable two-parameter affine model, $\mathbf{YDEV_{r,i} = \alpha_r \cdot YREF_{r,i} + t_r}$, where $YDEV_{r,i}$ is the obtained calibration signature of the predetermined calibration fluid of index i of the calibration group of index r, $YREF_{r,i}$ is the reference calibration signature of the predetermined calibration fluid of index i of the calibration group of index r, $\alpha_r$ is the value of a first adjustable multiplicative parameter of the affine model for the calibration group of index r and $t_r$ is the value of a second adjustable additive parameter of the affine model for the calibration group of index r.

7. The calibration method according to claims 2 and 6, wherein the correction (112) of the signature (YDEV) of said any fluid comprises the calculation YCOR = $(YDEV-t_{r̂})/\alpha_{r̂}$, where YDEV is the signature obtained by interaction of said any fluid with said at least one sensor (18) of the electronic device (10) to be calibrated, YCOR is the signature of said any fluid after correction and r̂ is the index of the selected calibration group.

8. The calibration method according to claim 6 or 7, wherein one of the first adjustable multiplicative parameter and the second adjustable additive parameter is independent of the calibration groups.

9. A computer program downloadable from a communications network and/or recorded on a computer-readable medium (22) comprising instructions that, when executed by a processing unit (24) of an electronic device for characterizing a fluid (10) to be calibrated, said processing unit (24) being capable of exchanging data with a memory (40) in which are stored:

- a plurality of reference calibration signatures ($YREF_{r,i}$) relating respectively to an interaction of a plurality of predetermined calibration fluids with at least one sensor of a reference electronic characterization device;
- a parametric model ($MOD(\alpha,t)$), with at least one adjustable parameter, of a drift of the electronic fluid characterization device (10) to be calibrated with respect to the reference electronic device;

lead the processing unit (24) to perform the following steps in interaction with the memory (40) and with at least one sensor (18) of the electronic fluid characterization device (10) to be calibrated:

- for each of the plurality of predetermined calibration fluids, obtaining (104) a calibration signature ($YDEV_{r,i}$) by interaction with said at least one sensor (18);
- determining (106) at least one value ($\alpha_r, t_r$) of said at least one adjustable parameter ($\alpha,t$)) by comparison of the obtained calibration signatures ($YDEV_{r,i}$) with the reference calibration signatures ($YREF_{r,i}$);

**characterized in that**, the predetermined calibration fluids being divided into a plurality of different calibration groups, the instructions are more specifically designed so that the determination (106) of said at least one value ($\alpha_r, t_r$) of said at least one adjustable parameter ($\alpha,t$) is carried out specifically for each calibration group.

10. A calibrated electronic device (10) for characterizing a fluid by providing a signature (YDEV) obtained from an electrical measurement signal (S), comprising:

- at least one sensor (18) designed to interact with the fluid;
- a transducer (20) designed to provide, in interaction with said at least one sensor (18), the electrical measurement signal (S);
- a memory (40, 44) for storing:

    • a parametric model ($MOD(\alpha,t)$), with at least one adjustable parameter ($\alpha,t$), of a drift of the calibrated electronic device (10) with respect to a reference electronic characterization device,
    • at least one value ($\alpha_r, t_r$) of said at least one

adjustable parameter ($\alpha,t$), calculated by prior calibration of the calibrated electronic device by comparison with the reference electronic device based on a plurality of predetermined calibration fluids; and

- a processor (24) for correcting the signature (YDEV) by applying said at least one adjustable parameter value;

**characterized in that**:

- the predetermined calibration fluids are divided into several different calibration groups and the memory stores at least one specific value ($\alpha_r, t_r$) of said at least one adjustable parameter ($\alpha,t$) for each of the calibration groups; and
- the processor (24) is more specifically programmed (46, 48) to select a calibration group to be associated with the fluid to be characterized based on its signature (YDEV), then to correct its signature using said at least one specific adjustable parameter value ($\alpha_r, t_r$) of the selected group.

[Fig. 1]

## Figure 1

[Fig. 2]

## Figure 2

[Fig. 3]

## Figure 3

[Fig. 4]

## Figure 4

[Fig. 5]

## *Figure 5*

[Fig. 6]

## *Figure 6*

[Fig. 7]

_Figure 7_

[Fig. 8]

_Figure 8_

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2020141281 A1

- WO 2022053690 A1

**Littérature non-brevet citée dans la description**

- **ZHANG et al.** On-line sensor calibration transfer among electronic nose instruments for monitoring volatile organic chemicals in indoor air quality. *Sensors and Actuators B: Chemicals*, 15 December 2011, vol. 160 (1), 899-909